# EUROPEAN PATENT SPECIFICATION

(11) **EP 1 875 869 B1**
(45) Date of publication and mention of the grant of the patent: **31.03.2010**
(21) Application number: 06116855.5
(22) Date of filing: 07.07.2006
(51) Int. Cl.: A61B 17/072, A61B 17/00

(54) **A deployment system for introducing a surgical instrument in a patients body**
Einführsystem für ein chirurgisches Instrument
Système de déploiement pour introduire un instrument de chirurgie dans le corps

(43) Date of publication of application: 09.01.2008
(73) Proprietor: ETHICON ENDO-SURGERY, INC., Cincinnati, OH 45242-2839 (US)
(72) Inventor: Pastorelli, Alessandro, I-00136, ROMA (IT); D'Arcangelo, Michele, I-00143, ROMA (IT); Bilotti, Federico, I-04011, APRILIA (Latina) (IT); Popovic, Drago, Sunrise beach, Queensland 4567 (AU)
(74) Representative: Leihkauf, Steffen Falk

(56) References cited:
- EP-A- 1 639 936
- WO-A1-03/015848
- WO-A2-2004/021867
- US-A1- 2002 068 935

## Description

The invention relates to a deployment system for introducing a surgical instrument in a patient's body.

With particular reference to a surgical stapling instrument, which can be used, e.g., in the diagnosis and/or therapy of pathologies of the lower gastrointestinal tract, there is a general need to introduce the surgical stapler deeply into the colon in order to reach the region of the pathology which needs to be treated.

A known surgical stapling instrument, disclosed in WO 01/91646 A1, has a staple fastening assembly located in the distal end region of the stapling instrument, a rigid shaft, and a handle extending from the shaft in the proximal end region of the stapling instrument. This known surgical stapling instrument can be used to excise tissue, e.g. polyps, and to stop bleeding virtually immediately. In a surgical procedure, the stapling instrument is introduced, e.g., into the anal canal and moved to the site of the tissue to be resected. The tissue to be excised can be pulled by means of a separate tissue grasping instrument into the area between an anvil and a cartridge device of the stapler, when the cartridge device and the anvil are in a spaced or open position. Afterwards, the anvil is moved relatively with respect to the cartridge device in order to clamp the tissue. When the cartridge device and the anvil have reached the closed position, the surgeon can "fire" the instrument, which means that the staples are driven out of the cartridge device, penetrate the tissue, whereupon the ends are bent by the anvil, and a knife or cutting edge is moved towards the anvil in order to remove the tissue. When the instrument is opened, the completely excised tissue can be safely removed from the patient's body together with or prior to removing the instrument itself.

Because of the rigidity and limited length of its insertion shaft, the operational range of the known surgical stapling instrument is restricted to an extent that it cannot be used e.g. to excise tissue of the anal canal remote from the rectum.

In order to obviate these drawbacks related to the insertion and deployment of the instrument, a surgical stapling device (disclosed in W02006/027014) has been proposed, in which the rigid insertion shaft has been replaced by a so called flexible "backbone" which flexibly connects a proximal handle and a distal staple fastening assembly. The flexible "backbone" is a kind of flexible shaft or flexible connection, for instance an endoscope which can be considerably long and enables the stapling instrument to be introduced, e.g. into the anal canal, and to be moved forward for a rather long distance to allow for the treatment of tissue at a site remote from the anus. During the introduction and advancement of the stapler, the flexible backbone adapts to the curvature of the intestine.

Even though the latter solution consents, from a purely mechanical point of view, a deeper introduction of the surgical instrument in the patients body, it is still not free from the risk of injuring or even perforating the delicate tissue of the endoscopic duct, e.g. the colonic wall of a patient. This is mainly due to the fact that, firstly the surgical stapling device, even though reduced to endoscopic dimensions, is relatively bulky compared with respect to the endoscope itself and with respect to the natural duct it needs to pass through and, secondly the instrument must pass through curved and tortuous passages of the natural duct thereby urging against the surrounding body tissue.

WO 03/015848 A, EP 1639936, US 2002/0068935 A, WO 2004/021867 A describe examples of movable couplings in surgical device deployment systems with EP 1 639 936 disclosing assemblies according to the preamble of claims 1 and 12.

In view of the drawbacks of the prior art deployment systems, it is the object of the present invention to provide a deployment system for introducing a surgical instrument in a patients body, for instance a system for delivering a surgical stapler through the endoscopic route, which permits a save introduction and removal of the instrument and minimizes the risk of trauma or perforation of the surrounding tissue, e.g. the colonic wall of a patient during a colonoscopy.

Within the general scope of the main object, it is a further aim of the present invention to provide a surgical instrument, in particular a surgical stapling device, having features such as to implement the deployment system of the present invention.

These and other problems are solved by a deployment system for introducing a surgical instrument in a patients body by means of an elongate insertion device which can be inserted in the patients body (by endoscopy, laparoscopy or open surgery), wherein the deployment system includes a coupling device connectable with the surgical instrument and movably connectable with the insertion device such that the surgical instrument can slide along the insertion device in a guided manner, wherein the coupling device includes one or more roller elements configured to rollably contact the insertion device.

Thereby, the coupling device of the deployment system can carry comparatively large and cumbersome instruments along the insertion device with very low friction, enabling the surgeon or the gastro-enterologist to deliver such instruments in a better controllable manner and reducing the risk of trauma of the tissue defining the access duct to the operational site. With particular reference to a surgical stapling device of the type described with reference to the prior art, thanks to the roller elements it becomes possible to introduce the stapling device deeply into the colon along a previously positioned colonoscope, thereby reducing the risk of accidental perforations of the colonic wall tissue.

In accordance with an important aspect of the invention, the coupling device of the delivery system comprises one or more roller bearings having a plurality of roller elements such as spherical, cylindrical or truncated cone shaped rollers, which are rotatably received in special roller seats or cavities of the bearing. In this context, it can be advantageous to provide a bearing having a substantially annular shape in order to embrace and rollably engage the insertion device substantially all around its outer periphery. Alternatively, it might be advantageous to provide a bearing having an arched shape open on one side and suitable to partially embrace and rollably engage the insertion device.

According to a yet further embodiment of the invention, an "internal" bearing might be provided, having the roller elements arranged on its outside and configured to contact and rollably engage the inside of a hollow or concave or tubular insertion device such as for instance a tubular protective sheath. Such a sheath might be necessary for the safe delivery of sharp edged instruments, in order to protect the endoscopic access duct, or very sensible and easily damageable surgical or diagnostic instruments, in order to protect them from a direct contact with the body tissue during delivery.

In accordance with a further aspect of the invention, the coupling device comprises a connecting portion which is configured to allow the coupling device to be detachably connected with a surgical instrument, preferably by snap-fit. Thereby allowing the surgeon to easily assemble the surgical instrument with the coupling device and attaching it to a distal end portion of the insertion device, disposed outside the body of the patient. Moreover, a detachable connection between the coupling device and the surgical instrument allows the substitution of different instruments to be carried by the same coupling device or the use of different insertion guides and, hence, different coupling devices with the same surgical instrument.

The surgical instrument or more generally spoken its actuating portion (e.g. a staple fastening assembly of a surgical stapling device) which is rollably guided along the insertion device has preferably a substantially elongate shape in approximate alignment with the longitudinal direction of the insertion device. An improvement of the stability of the relative positioning between the actuating portion and the insertion device can be obtained by connecting the actuating portion with at least two roller bearings, both engaging the insertion device and preferably arranged near two opposite ends of the actuating portion with reference to its longitudinal extension.

According to a yet further aspect of the invention, the actuating portion of the surgical instrument defines a sliding surface with an approximately complementary shape to a corresponding surface of the insertion device which is destined to slidably contact the sliding surface of the actuating portion. In this case, the roller elements of the coupling device are arranged substantially opposite the sliding surface in such a way that the surgical instrument engages the insertion device slidably and rollably. This solution represents a synergistic compromise which reconciles the need of structural simplicity and reduced frictional resistance. Moreover, the at least approximate shape complementarity of the contacting sliding surfaces of the insertion device and the surgical instrument together with the embracing roller bearing enable the surgical instrument to be positioned in intimate contact with the insertion instrument.

The deployment system of the present invention will be preferentially used within endoscopic settings, wherein the elongate insertion device comprises advantageously a flexibly adjustable endoscope, e.g. a colonoscope, but also laparoscopic or open surgery approaches are contemplated and can be advantageous in all those situations that require a low friction translation of a surgical instrument along a previously deployed guide ore insertion device and in which, for some reason, the endoscopic approach is not adopted.

In order to move the surgical instrument in a controlled manner along the insertion device, it is advantageous to provide shifting means connected with the surgical instrument and configured to transmit pushing and/or pulling movements to the instrument. During the surgical procedure, these shifting means which can be embodied by preferably flexible compression and/or tension rods, are advantageously guided along the insertion device and extend from the surgical instrument proximally outside the patient's body in order to be directly operable by the surgeon.

According an embodiment of the invention, the shifting means are also shiftably or slidably linked to the insertion device, preferably by one or more coupling devices including roller elements, thereby constraining the movement of the entire system to the desired guide path and at the same time reducing its frictional resistance to a minimum.

In accordance with a preferred embodiment of the invention, the shifting means are distally connected with the surgical instrument or, more generally, with an actuating portion thereof (e.g. a staple fastening assembly of a surgical stapling device), and a proximal end of the shifting means is connected with a handle including an actuating mechanism of the surgical instrument, wherein the same shifting means that transmit the deployment movements to the actuating portion defines one or more internal channels which receive means for the transmission of the actuating movements generated by the handle actuating mechanism to the actuating portion.

Advantageously, the deployment system of the present invention provides blocking means or breaking means suitable to block the surgical instrument or the coupling device with respect to the insertion device in order to fixate at least temporarily their relative position. Such fixation of the surgical instrument to the insertion device allows the instrument to be moved together with and by the insertion device, for instance in situations in which the movement capabilities of the insertion device (e.g. an endoscope) are better than those of the surgical instrument. The blocking means are preferably activated in response to a blocking force, which can be generated by the handle actuating mechanism and transmitted to the blocking means by special blocking force transmitting means preferably received in the above mentioned shifting means. This, in turn contributes to limiting the overall encumbrance of the deployment system such that the risk of accidental interference with the surrounding body tissue is further reduced.

These and other details and advantages of the present invention shall be made apparent from the accompanying drawings and the description thereof, which illustrate embodiments of the invention and, together with the general description of the invention given above, and the detailed description of the invention given below, serve to explain the principles of the present invention.
- Figure 1 is a proximal isometric view of a surgical stapling device with a deployment system according to a first embodiment of the invention;
- Figure 2 is a distal view of the surgical stapling device of figure 1;
- Figure 3 is a distal isometric view of the surgical stapling device of figure 1 coupled with an insertion device;
- Figure 4 is a distal isometric view of a surgical stapling device with a deployment system according to a second embodiment of the invention in a closed configuration and coupled to an insertion device;
- Figure 5 is a distal isometric view of the surgical stapling device of figure 4 in an opened configuration and coupled to an insertion device;
- Figure 6 shows a distal isometric view of the surgical stapling device of figure 4 in a closed configuration and a insertion device approximating towards a coupling device of the surgical stapling device;
- Figure 7 shows a distal isometric view of the surgical stapling device of figure 4 in a closed configuration and the insertion device engaging a proximal part of the coupling device of the surgical stapling device;
- Figure 8 shows a distal isometric view of the surgical stapling device of figure 4 in a closed configuration and the insertion device fully engaging a proximal and distal part of the coupling device of the surgical stapling device;
- Figure 9 is a top view of the situation shown in figure 8;
- Figures 10 and 11 are schematic partially sectional views showing the procedure of deploying the surgical stapling device in a patients body;
- Figures 12 and 13 are schematic isometric overall views of a surgical stapling device implementing a deployment system according to two embodiments of the invention.

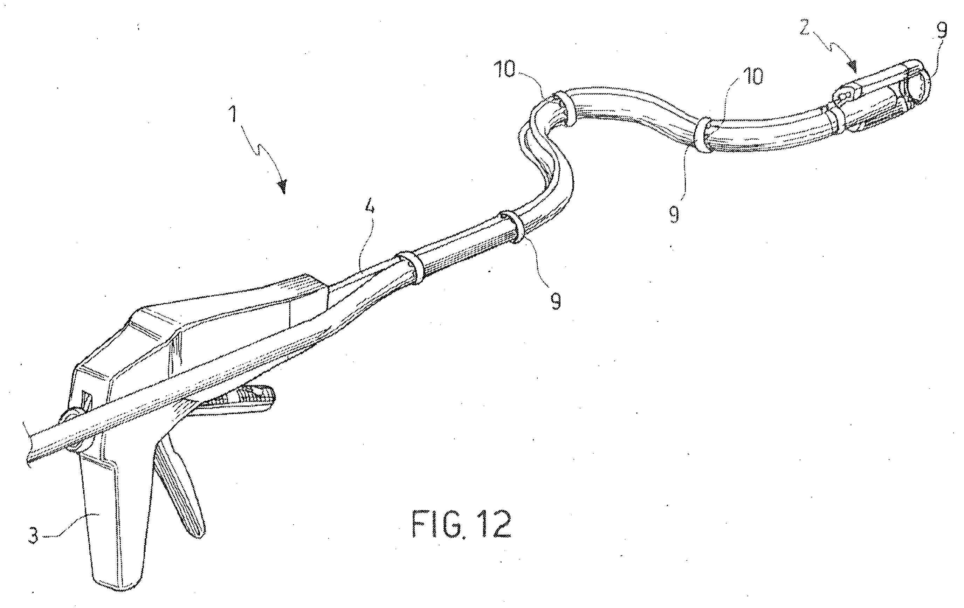

Turning to the figures, fig. 12 is an isometric overall view of a surgical stapling instrument 1 according to a first embodiment. The stapling instrument 1 comprises, in its distal end region, a staple fastening assembly 2 and, in its proximal end region, a handle 3. The handle 3 and the staple fastening assembly 2 are connected via two elongate flexible force transmitters 4, 5 used for transmitting forces from the handle 3 to the staple fastening assembly 2 in order to perform the functions of the stapling instrument 1.

The main components of the staple fastening assembly 2 are a cartridge device 6, which contains several curved open rows of staples as well as a knife or cutting edge, and a curved anvil 7, which has a staple forming face and is adapted to cooperate with the cartridge device to form the ends of the staples expelled from the cartridge device when stapling instrument 1 is "fired".

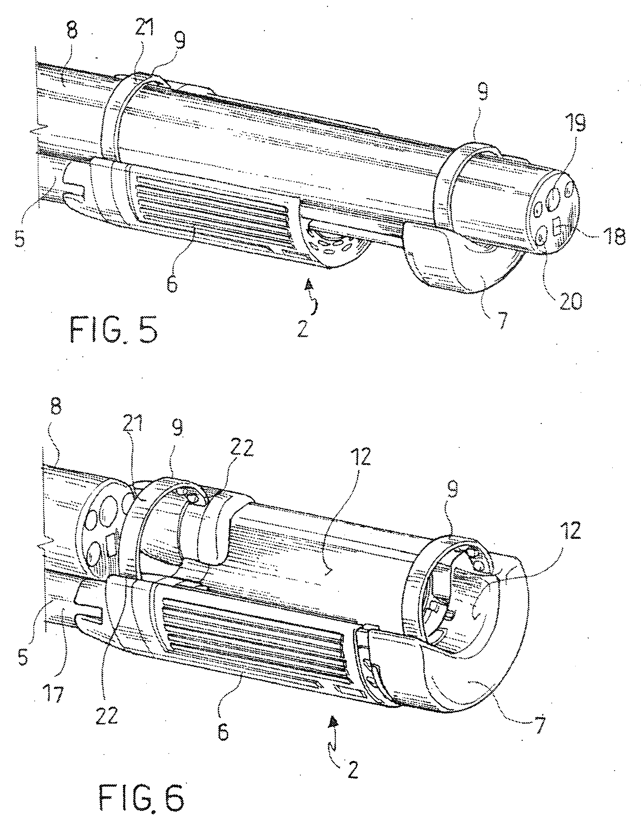

The anvil 7 can be moved with respect to the cartridge device 6 in a parallel relationship, i.e. in a direction parallel to a longitudinal axis of the staple fastening assembly 2. In the view of Fig. 5, the anvil 7 is spaced apart from the cartridge device 6, while in Fig. 6, the same anvil 7 has been entirely moved towards the cartridge device 6. The mechanism and its components of the stapling instrument 1 used for moving anvil 7 relative to cartridge device 6 are generally called moving device, whereas the mechanism and the components used for advancing the staples are generally called staple driving device.

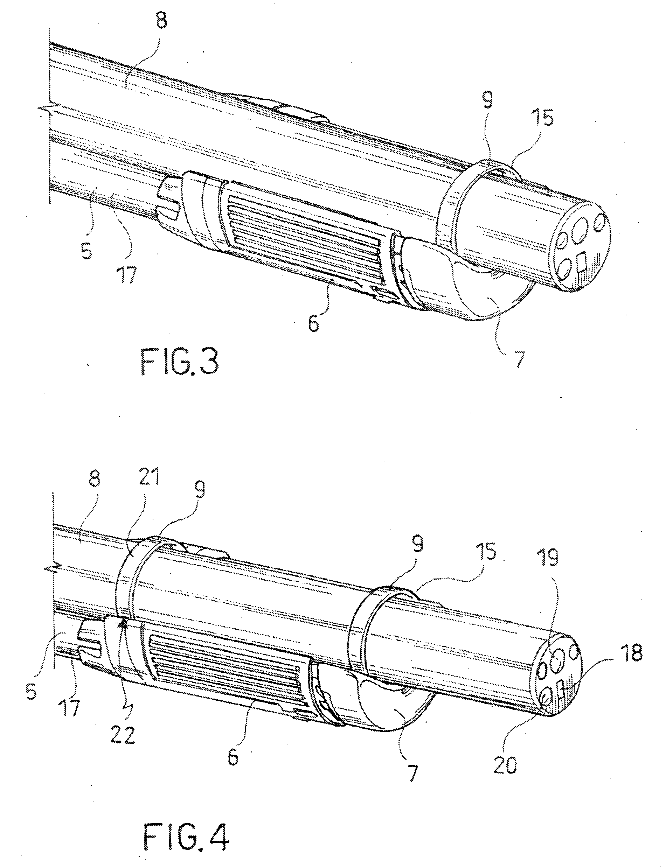

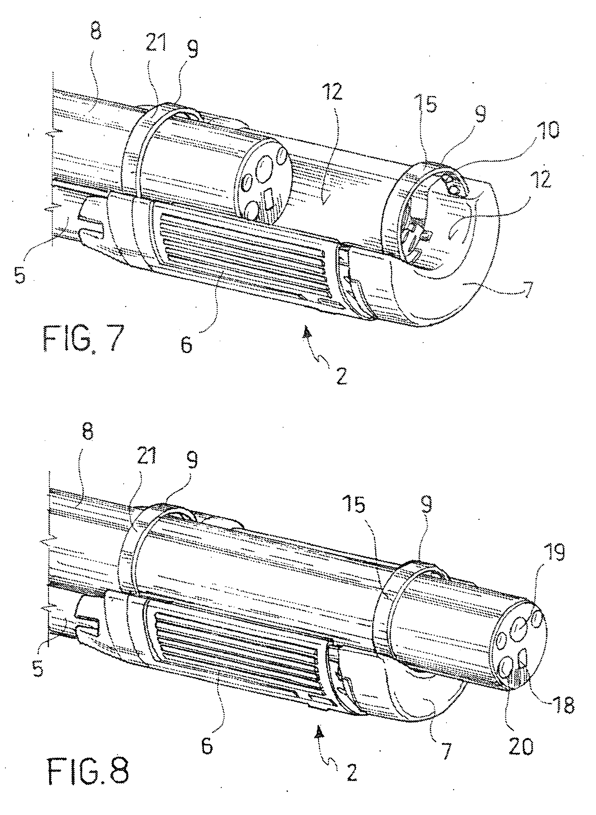

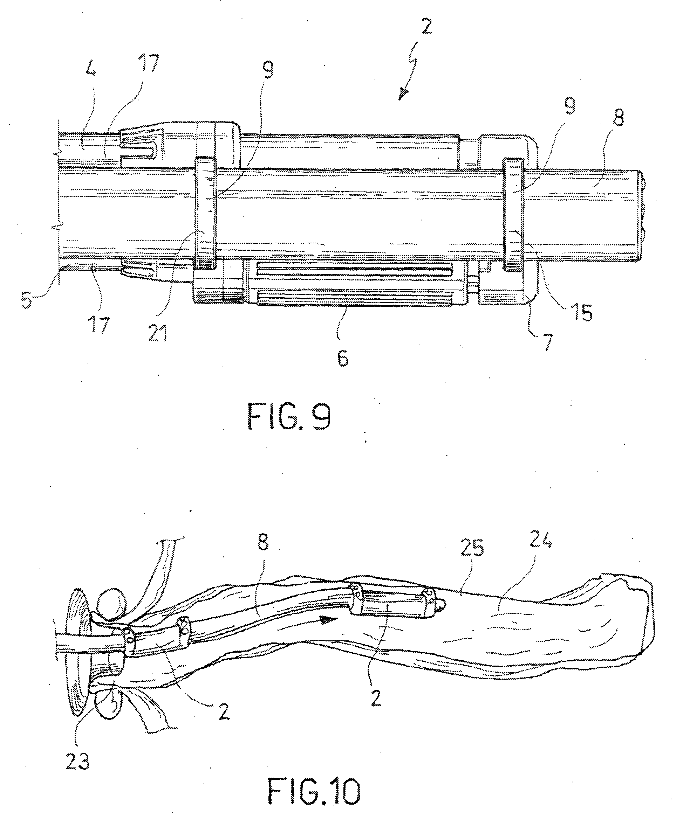

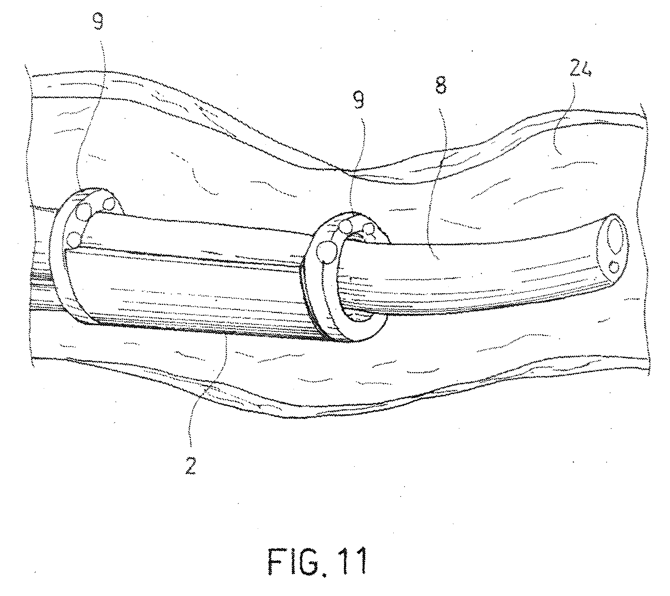

Since the present invention concentrates primarily on the deployment system for a surgical instrument and a surgical instrument embodying such a deployment system, for the sake of simplicity, in the present description the above mentioned moving device and staple driving device will be referred to as a whole with the term "actuating mechanism" of the instrument 1. As the detailed description of one possible embodiment of such an actuating mechanism is concerned, such a description is disclosed in the applicant's co-pending international application WO 2006/027014, figures 4 to 15 and the

In order to introduce the staple fastening assembly 2 of the stapling device in the lower gastrintestinal tract of a patient, an elongate insertion device, for instance a flexibly adjustable colonoscope 8 is provided and transanally introduced in the colon until it reaches the excision site. After positioning of the colonoscope 8, the staple fastening assembly 2 of the surgical stapling device 1 is shiftably or slidably coupled to a proximal portion of the colonoscope 8 reaching out of the patient's body and distally pushed along the colonoscope until it reaches the excision site. The sliding connection between the staple fastening assembly 2 and the colonoscope 8 is obtained by a coupling device 9 which can be detachably connected with the staple fastening assembly 2 and slidably coupled to the colonoscope 8. The coupling device 9 includes one or more roller elements 10 which are configured to rollably contact an outer surface of the colonoscope 8, thereby reducing the frictional resistance between the coupling device 9 and the colonoscope 8.

In particular, the coupling device 9 comprises at least one roller bearing with a plurality of preferably spherical or, alternatively, cylindrical or truncated cone shaped roller elements 10 which are captively received in special seats 11 defined in the coupling device or held by pins (not shown in the figures) which allow the roller elements 10 to rotate around respective pin longitudinal axes.

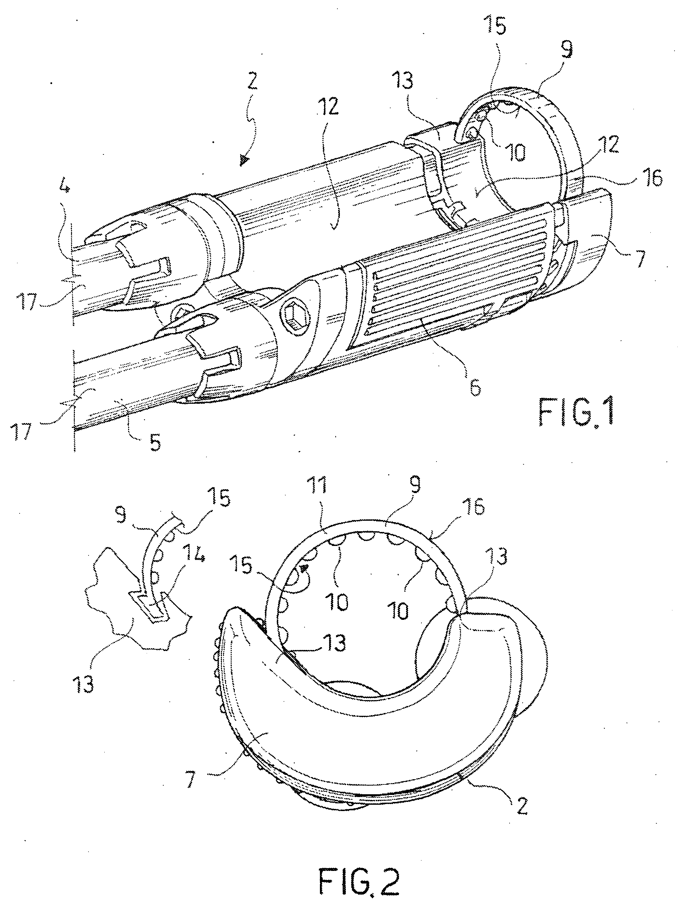

In accordance with the embodiment shown in figures 1 to 3, the cartridge device 6 and the anvil 7 of the staple fastening assembly 2 have a generally elongate rectangular arc-like shape which enables an easy access to the working area between cartridge device 6 and anvil 7 and defines a concave sliding surface 12 approximately complementary with the shape of a part of the outer surface of the colonoscope 8. In particular, the colonoscope 8 has a substantially cylindrical (curved) outer surface and the sliding surface 12 of the staple fastening assembly 2 has accordingly the approximate shape of a sector of a cylinder, preferably a halve cylinder. The staple fastening assembly 2 comprises a distal connecting portion with two opposite snap engagement seats 13 arranged near the distal end of the assembly 2 and configured to detachably engage corresponding snap engagement members 14 of an arch-shaped, approximately semi-circular distal ball bearing 15. The snap-engagement seats 13 and the corresponding snap-engagement members 14 may be embodied by an elastically snap engaging hook-catch system as schematically shown in figure 2 or by any suitable equivalent or alternative connecting system.

The distal ball bearing 15 comprises a plurality of spherical roller members 10 which are arranged (when the ball bearing 15 is mounted on the staple fastening assembly 2) opposite the concave sliding surface 12 and define, together with the approximately semi-cylindrical sliding surface 12 a ring structure which embraces the colonoscope 8 thereby engaging its outer surface both slidably and rollably. In accordance with the embodiment shown in the figures, the snap engagement seats 13 for the distal ball bearing 15 are formed in the anvil 7.

The distal ball bearing 15 comprises a substantially smooth and continuous approximately semi-cylindrical cover wall 16 arranged radially outside the roller elements and covering them from an outside, distally and proximally, thereby preventing exposure of the roller elements 10 to the surrounding body tissue.

Each of the two force transmitters 4, 5 comprise a preferably flexible but longitudinally inextensible tube 17 which forms a longitudinal force transmitting compression/tension rod for transmitting pushing and/or pulling movements from outside the patient's body to the staple fastening assembly 2 in order to make the latter slide along the colonoscope 8. During the surgical procedure, the tubes 17 are guided closely along the colonoscope 8, for instance by means of shiftable or slidable connectors, preferably by one or more coupling devices including roller elements similar to the coupling device 9 which is attached to the staple fastener assembly 2. This enables the deployment system to constrain the movements of the entire surgical instrument along the desired guide path and to reduce its frictional resistance to a minimum.

As already mentioned, a distal end of the inextensible tubes 17 is connected with a proximal end portion of the staple fastening assembly 2 and a proximal end of the inextensible tubes 17 is connected with the handle 3 of the surgical stapling device 1 such that a movement of the handle 3 along an extra-corporeal proximal portion of the colonoscope 8 causes a corresponding movement of the staple fastening assembly 2 along an intra-corporeal distal portion of the colonoscope 8.

In addition to their function of shifting the staple fastening assembly 2 along the colonoscope 8, the inextensible tubes 17 define internally one or more channels which are suitable to house transmitting means for the transmission of the actuating forces of the surgical stapling device 1. These transmitting means are preferably translating or rotary transmitting rods for the transmission of anvil moving forces, staple driving forces and cutting forces generated by the handle actuating mechanism.

Even though the surgeon may continuously actively control the position of the staple fastening assembly by manually controlling its movement along the colonoscope, it can be advantageous to provide a blocking means or braking means suitable to block the staple fastening assembly 2 or the coupling device 9 with respect to the colonoscope 8. Such fixation of the staple fastening assembly enables the surgeon to move the latter together with the colonoscope. This can be very helpful in situations where the motive capabilities of the colonoscope are better and more precise than that of the staple fastening assembly. The blocking means can comprise clamping mechanisms with friction pads or forceps configured to frictionally engage the outer surface of the colonoscope in response to a blocking or braking force, which can be generated by the handle actuating mechanism and transmitted to the blocking means by special blocking force transmitting rods or wires which can be arranged inside the tubes 17.

The colonoscope 8 shown in the figures is a commercially available endoscope which need not be modified in order to be used for deploying the stapling device 1. The endoscope 8 can be flexibly curved during its introduction in the natural duct (colon) of the patient and, once brought in its final position, it provides structural strength to the surgical instrument 1 and to the endoscopic duct during the rollable insertion of the instrument.

Fig. 3 illustrates the arrangement of the distal portion of the endoscope 8 and the staple fastening assembly 2. The distal end face of endoscope 8 has two openings, one for observational optics 18 and one for a light source 15. Preferably, the endoscope includes also a working channel 20 which allows the introduction of a further surgical instrument (e.g. an endoscopic grasper having a flexible shaft) to the site of surgery, i.e. to the area of the staple fastening assembly 2.

Fig. 4 to 8 show a further embodiment of the invention, wherein like numerals denote like components which have already been described in relation with the first embodiment. In this second embodiment, the coupling device comprises one distal roller bearing 15 connected with the distal connecting portion 13 of the anvil 7 and an additional proximal roller bearing 21 which can be configured as previously described in relation with the distal bearing 15. The proximal roller bearing 21 is also advantageously detachably connectable with a proximal connecting portion, e.g. with two opposite snap engagement seats 22 formed in a proximal end region of the cartridge device 6 and spaced apart from the distal bearing 15 in a longitudinal direction of the staple fastening assembly 2. Both roller bearings 15 and 21 engage the outer surface of the endoscope 8 in rollable contact thereby providing a particularly stable and low frictional sliding engagement between the staple fastening assembly and the endoscope 8.

The deployment system according to the present invention and the endoscopic stapling device embodying the deployment system can be advantageously used for instance for the treatment of pathologies in the colon. In a first step of such an endoscopic intervention, the endoscope 8 is introduced through the rectum 23 and anal canal of a patient until it reaches the portion of the colon 25 which needs to be treaded (excision site 24). After the placement of the endoscope 8, the staple fastening assembly 2 is connected with the proximal extra-corporeal portion of the endoscope 8 in a slidable manner, wherein the coupling device with its roller elements provides a rollable contact between the staple fastening assembly 2 and the outer surface of the endoscope 8 and, preferably, a complete embracement of the endoscope 8 such that the staple fastening assembly 2 is tightly approximated to it.

The staple fastening assembly 2 rollably connected with the endoscope is transanally introduced in the anal canal and slided along the endoscope until it reaches the excision site 25. This sliding movement is brought about by a pushing force of the handle transmitted by the inextensible tubes 17 to the staple fastening assembly 2.

After deployment of the staple fastening assembly 2, an endoscopic grasper is distally advanced through the working channel 20 of the endoscope 8 until it exits from its distal end opening. The lesion, e.g. a polyp or a tumor, can now be grasped by the endoscopic grasper and dragged inside the space between the anvil and the cartridge device of the staple fastening assembly 2. Upon operation of the actuating mechanism of the stapling device, the anvil is moved towards the cartridge device clamping the tissue between them. After clamping of the tissue, the stapler is "fired" and, hence, the staples are driven in the tissue and the lesion is excised by the knife of the stapling device. After completion of the excision, the staple fastening assembly 2 can be removed from the patients body by pulling it proximately along the endoscope or, alternatively, it can be withdrawn together with the endoscope. The excised sample of pathologic tissue can be removed in proximal direction through the working channel of the endoscope or, if it is too bulky for passing the working channel, it can be withdrawn together with the endoscope.

While the present invention has been illustrated by description of several embodiments and while the illustrative embodiments have been described in considerable detail, it is not the intention to restrict or in any way limit the scope of the appended claims to such detail. Additional advantages and modifications may readily appear to those skilled in the art.

For instance, the deployment system can be equally well used for the introduction and deployment of surgical or diagnostic instruments different from endoscopic staplers. Accordingly, the surgical or diagnostic instrument comprising a coupling portion with roller elements in order to implement the deployment system of the invention is not limited to the described example. The deployment system can be advantageously integrated in surgical and diagnostic instruments different from an endoscopic stapler.

With regard to the insertion device, such device is not limited to the endoscopes which have been described in some detail. Instead or additionally, the insertion device may comprise a laparoscope, a flexible or rigid, curved or straight, elongate shaft or hollow sheath, wherein the roller elements of the coupling device can contact the insertion device from an outer side or internally and the surgical instrument itself can be rollably slided along an outside or through an internal channel of the insertion device.

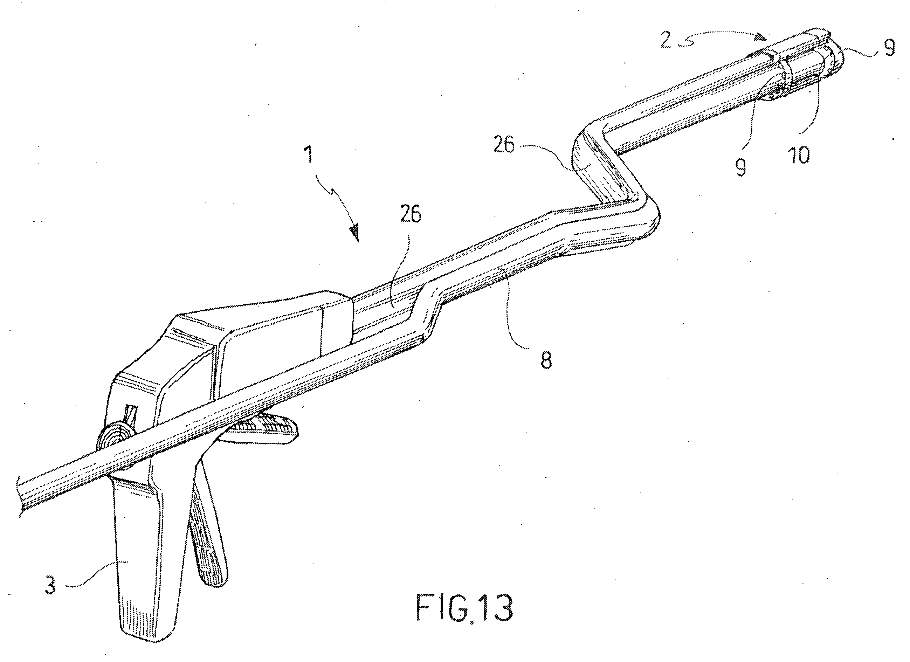

Finally, the shifting means, i.e. the inextensible tubes 17 need not be two and need not be tubular. They could be replaced by a pulling cable mechanism or by a single shifting rod, as can be seen for instance in fig. 13, where all actuating force transmitters are housed in a single flexible shifting tube 26 guided along the insertion device, which can be an endoscope or a different elongate guiding structure.

In this embodiment, the single flexible shifting tube 26 defines internally two or more longitudinal channels for guiding rotary or translating rods of the force transmitters of the moving device and of the staple driving device.

## Claims

1. A deployment system for introducing a surgical instrument (2) in a patients body by means of an elongate insertion device (8) which can be inserted in the patients body, wherein the deployment system, comprises a coupling device (9, 15, 21) connectable with the surgical instrument (2) and movably connectable with the insertion device (8) such that the surgical instrument (2) can slide along the insertion device (8) in a guided manner, **characterized in that** the coupling device (9) includes one or more roller elements (10) configured to rollably contact the insertion device (6).

2. A deployment system according to claim 1, wherein the coupling device (9) comprises one or more roller bearings (15, 21) having a plurality of roller elements (10),

3. A deployment system according to claim 2, wherein the roller bearing (15, 21) has a substantially annular shape suitable to embrace and rollably engage the insertion device (8) substantially all around its outer periphery.

4. A deployment system according to claim 2, wherein the roller bearing (15, 21) has an arched shape suitable to partially embrace and rollably engage the insertion device (8).

5. A deployment system according to any one of the preceding claims, wherein the coupling device (9) comprises a connecting portion (14) which is configured to detachably connect with the surgical instrument (2).

6. A deployment system according to the preceding claim, wherein the connecting portion (14) is configured to snap-engage a corresponding connecting portion (13, 22) of the surgical instrument (2).

7. A deployment system according to any one of the preceding claims, comprising said coupling device (9) and said surgical instrument (2) wherein the surgical instrument (2) is connectable with at least two roller bearings (15, 21), both suitable to engage the insertion device (8) and spaced to one another in a longitudinal direction of the surgical instrument (2).

8. A deployment system according to the preceding claim, wherein the surgical instrument (2) defines a sliding surface (12) having a shape approximately complementary with the shape of a corresponding sliding surface of the insertion device (8), wherein the roller elements (10) of the coupling device (9, 15, 21) are arranged substantially opposite the sliding surface (12) in such a way that the surgical instrument (1) engages the insertion device (8) slidably and rollably,

9. A deployment system according to any one of the preceding claims, comprising shifting means (17) connectable with the surgical instrument (2) and configured to transmit pushing and/or pulling movements to the instrument (2).

10. A deployment system according to the preceding claim, wherein the shifting means (17) are connected distally with the surgical instrument (20) and proximally with a handle (3) thereof, said handle including an actuating mechanism of the surgical instrument and said shifting means (17) comprising an inextensible tube (17) defining one or more internal channels which house means for the transmission of actuating movements generated by the handle actuating mechanism.

11. A deployment system according to any one of the preceding claims, comprising said elongate insertion device (8), wherein said elongate insertion device (8) is an endoscope.

12. An endoscopic stapling device (1) comprising:
- a staple fastening assembly (2) suitable to resect a tissue sample and suture the site of excision;
- a coupling device (9, 15, 21) connectable with the staple fastening assembly (2) and movably connectable with an elongate insertion device (8) such that the staple fastening assembly (2) can slide along the insertion device (8) in a guided manner, **characterized in that** the coupling device (9) includes one or more roller elements (10) configured to rollably contact the insertion device (8).

## Patentansprüche

1. Einsatzsystem zum Einführen eines chirurgischen Instruments (2) in den Körper eines Patienten mittels einer länglichen Einführungsvorrichtung (8), die in den Körper des Patienten eingeführt werden kann, wobei das Einsatzsystem eine Kopplungsvorrichtung (9, 15, 21) umfasst, die mit dem chirurgischen Instrument (2) verbindbar ist und mit der Einführungsvorrichtung (8) beweglich verbindbar ist, so dass das chirurgische Instrument (2) entlang der Einführungsvorrichtung (8) in einer geführten Weise gleiten kann,
**dadurch gekennzeichnet, dass** die Kopplungsvorrichtung (9) einen oder mehrere Wälzelemente (10) umfasst, die dazu konfiguriert sind, rollbar mit der Einführungsvorrichtung (8) in Kontakt zu stehen.

2. Einsatzsystem nach Anspruch 1, wobei die Kopplungsvorrichtung (9) ein oder mehrere Wälzlager (15, 21) umfasst, die eine Mehrzahl von Wälzelementen (10) aufweisen.

3. Einsatzsystem nach Anspruch 2, wobei das Wälzlager (15, 21) eine im Wesentlichen ringförmige Form aufweist, die dazu geeignet ist, die Einführungsvorrichtung (8) zu umfassen und rollbar mit ihr im Wesentlichen um ihren gesamten Außenumfang in Eingriff zu stehen.

4. Einsatzsystem nach Anspruch 2, wobei das Wälzlager (15, 21) eine bogenförmige Form aufweist, die dazu geeignet ist, die Einführungsvorrichtung (8) teilweise zu umfassen und rollbar mit ihr in Eingriff zu stehen.

5. Einsatzsystem nach einem der vorhergehenden Ansprüche, wobei die Kopplungsvorrichtung (9) einen verbindenden Abschnitt (14) umfasst, der dazu konfiguriert ist, sich abtrennbar mit dem chirurgischen Instrument (2) zu verbinden.

6. Einsatzsystem nach dem vorhergehenden Anspruch, wobei der verbindende Abschnitt (14) dazu konfiguriert ist, mit einem entsprechenden verbindenden Abschnitt (13, 22) des chirgurgischen Instruments (2) in Schnappeingriff zu stehen.

7. Einsatzsystem nach einem der vorhergehenden Ansprüche, umfassend die Kopplungsvorrichtung (9) und das chirurgische Instrument (2), wobei das chirurgische Instrument (2) mit wenigstens zwei Wälzlagern (15, 21) verbindbar ist, die beide dazu geeignet sind, mit der Einführungsvorrichtung (8) in Eingriff zu stehen und mit Abstand voneinander in eine Längsrichtung des chirurgischen Instruments (2) angeordnet sind.

8. Einsatzsystem nach dem vorhergehenden Anspruch, wobei das chirurgische Instrument (2) eine Gleitoberfläche (12) definiert, die eine Form aufweist, die annähernd komplementär zu der Form einer entsprechenden Gleitoberfläche der Einführungsvorrichtung (8) ist, wobei die Wälzelemente (10) der Kopplungsvorrichtung (9, 15, 21) im Wesentlichen gegenüber der Gleitoberfläche (12) so angeordnet sind, dass das chirurgische Instrument (1) mit der Einführungsvorrichtung (8) gleitbar und rollbar in Eingriff steht.

9. Einsatzsystem nach einem der vorhergehenden Ansprüche, umfassend Schiebemittel (17), die mit dem chirurgischen Instrument (2) verbindbar sind und dazu konfiguriert sind, Schiebe- oder/ und Ziehbewegungen auf das Instrument (2) zu übertragen.

10. Einsatzsystem nach dem vorhergehenden Anspruch, wobei die Schiebemittel (17) distal mit dem chirurgischen Instrument (20) und proximal mit einem Griff (3) davon verbunden sind, wobei der Griff einen Stellmechanismus des chirurgischen Instruments umfasst und die Schiebemittel (17) eine nicht ausdehnbare Röhre (17) umfassen, die einen oder mehrere Innenkanäle definiert, die Mittel zur Übertragung von Stellbewegungen aufnehmen, die durch den Griff-Stellmechanismus erzeugt werden.

11. Einsatzsystem nach einem der vorhergehenden Ansprüche, umfassend die längliche Einführungsvorrichtung (8), wobei die längliche Einführungsvorrichtung (8) ein Endoskop ist.

12. Endoskopische Klammervorrichtung (1), umfassend:
- eine Klammerbefestigungsanordnung (2), die dazu geeignet ist, eine Gewebeprobe zu entnehmen und die Entnahmestelle zu nähen;
- eine Kopplungsvorrichtung (9, 15, 21), die mit der Klammerbefestigungsanordnung (2) verbindbar ist und so mit einer länglichen Einführungsvorrichtung (8) beweglich verbindbar ist, dass die Klammerbefestigungsanordnung (2) in einer geführten Weise entlang der Einführungsvorrichtung (8) gleiten kann,
**dadurch gekennzeichnet, dass** die Kopplungsvorrichtung (9) ein oder mehrere Wälzelemente (10) umfasst, die dazu konfiguriert sind, rollbar mit der Einführungsvorrichtung (8) in Kontakt zu stehen.

## Revendications

1. Système de déploiement pour introduire un instrument chirurgical (2) dans le corps d'un patient au moyen d'un dispositif d'insertion allongé (8) qui peut être inséré dans le corps du patient, dans lequel le système de déploiement comprend un dispositif de couplage (9, 15, 21) capable d'être connecté à l'instrument chirurgical (2) et d'être connecté de façon mobile avec le dispositif d'insertion (8) de telle façon que l'instrument chirurgical (2) peut coulisser le long du dispositif d'insertion (8) d'une manière guidée, **caractérisé en ce que** le dispositif de couplage (9) inclut un ou plusieurs éléments de roulement (10) configurés pour venir en contact en roulement contre le dispositif d'insertion (8).

2. Système de déploiement selon la revendication 1, dans lequel le dispositif de couplage (9) comprend un ou plusieurs paliers à roulement (15, 21) ayant une pluralité d'éléments de roulement (10).

3. Système de déploiement selon la revendication 2, dans lequel le palier à roulement (15, 21) a une forme sensiblement annulaire convenable pour enserrer et engager en roulement le dispositif d'insertion (8) sensiblement tout autour de sa périphérie extérieure.

4. Système de déploiement selon la revendication 2, dans lequel le palier à roulement (15, 21) a une forme arquée convenable pour enserrer partiellement et engager en roulement le dispositif d'insertion (8).

5. Système de déploiement selon l'une quelconque des revendications précédentes, dans lequel le dispositif de couplage (9) comprend une portion de connexion (14) qui est configurée pour être connectée de façon détachable avec l'instrument chirurgical (2).

6. Système de déploiement selon la revendication précédente, dans lequel la portion de connexion (14) est configurée pour engager en encliquetage une portion de connexion correspondante (13, 22) de l'instrument chirurgical (2).

7. Système de déploiement selon l'une quelconque des revendications précédentes, comprenant ledit dispositif de couplage (9) et ledit instrument chirurgical (2), dans lequel l'instrument chirurgical (2) peut être connecté avec au moins deux paliers à roulement (15, 21), qui conviennent tous les deux pour venir engager le dispositif d'insertion (8) et sont espacés l'un de l'autre dans une direction longitudinale de l'instrument chirurgical (2).

8. Système de déploiement selon la revendication précédente, dans lequel l'instrument chirurgical (2) définit une surface de coulissement (12) ayant une forme approximativement complémentaire de la forme d'une surface de coulissement correspondante du dispositif d'insertion (8), dans lequel les éléments de roulement (10) du dispositif de couplage (9, 15, 21) sont agencés sensiblement à l'opposé de la surface de coulissement (12) d'une manière telle que l'instrument chirurgical (1) engage le dispositif d'insertion (8) en coulissement et en roulement.

9. Système de déploiement selon l'une quelconque des revendications précédentes, comprenant des moyens de déplacement (17) capables d'être connectés à l'instrument chirurgical (2) et configurés pour transmettre des mouvements de poussée et/ou de traction à l'instrument (2).

10. Système de déploiement selon la revendication précédente, dans lequel les moyens de déplacement (17) sont connectés en position distale à l'instrument chirurgical (20) et en position proximale à une poignée (3) de celui-ci, ladite poignée incluant un mécanisme d'actionnement de l'instrument chirurgical, et lesdits moyens de déplacement (17) comprenant un tube inextensible (17) définissant un ou plusieurs canaux internes qui abritent des moyens pour la transmission de mouvements d'actionnement engendrés par le mécanisme d'actionnement de la poignée.

11. Système de déploiement selon l'une quelconque des revendications précédentes, comprenant ledit dispositif d'insertion allongé (8), dans lequel ledit dispositif d'insertion allongé (8) est un endoscope.

12. Dispositif de pose d'agrafe endoscopique (1), comprenant :
- un ensemble de fixation d'agrafe (2) convenable pour faire une résection d'un échantillon de tissu et pour suturer le site d'excision ;
- un dispositif de couplage (9, 15, 21) capable d'être connecté à l'ensemble de fixation d'agrafe (2) et capable d'être connecté de façon mobile avec un dispositif d'insertion allongé (8), de telle façon que l'ensemble de fixation d'agrafe (2) peut coulisser le long du dispositif d'insertion (8) d'une manière guidée, **caractérisé en ce que** le dispositif de couplage (9) inclut un ou plusieurs éléments de roulement (10) configurés pour venir en contact en roulement contre le dispositif d'insertion (8).
